# EUROPEAN PATENT SPECIFICATION

(11) **EP 2 729 139 B1**
(45) Date of publication and mention of the grant of the patent: **25.11.2020**
(21) Application number: 12808161.9
(22) Date of filing: 04.07.2012
(51) Int. Cl.: A61K 31/165, A61K 36/67, A61K 36/81, A61P 11/14, A61K 9/48, A23L 2/52, A23L 33/105

(54) **Cough reducing product**
Produkt zur Linderung von Husten
Produit pour réduire la toux

(30) Priority: 07.07.2011 US 201161571874 P
(43) Date of publication of application: 14.05.2014
(73) Proprietor: Millqvist, Eva, 54105 Skövde (SE)
(72) Inventor: Millqvist, Eva, 54105 Skövde (SE)
(74) Representative: Barker Brettell Sweden AB
(86) International application number: PCT/SE2012/050781
(87) International publication number: WO 2013/006135

(56) References cited:
- WO-A2-2008/002514
- WO-A2-2011/061330
- CN-A- 1 640 479
- CN-A- 101 731 573
- DE-A1- 10 317 843
- DE-A1-102004 063 363
- US-A- 5 134 166
- US-A- 5 134 166
- US-A- 5 431 914
- US-A- 5 994 407
- US-A- 5 994 407
- US-A1- 2004 054 337
- US-A1- 2008 241 290
- US-A1- 2009 093 446
- EVA MILLQVIST: "The airway sensory hyperreactivity syndrome", PULMORNARY PHARMACOLOGY & THERAPEUTICS, ACADEMIC PRESS, GB, vol. 24, no. 3, 2 October 2010 (2010-10-02), pages 263-266, XP028221380, ISSN: 1094-5539, DOI: 10.1016/J.PUPT.2010.10.001 [retrieved on 2010-10-10]
- MORICE A H ET AL: "Cough Hypersensitivity Syndrome: A Distinct Clinical Entity", LUNG, SPRINGER-VERLAG, US, vol. 189, no. 1, 14 December 2010 (2010-12-14), pages 73-79, XP019882921, ISSN: 1432-1750, DOI: 10.1007/S00408-010-9272-1
- MARABINI, S. ET AL.: 'Beneficial effects of intranasal applications of capsaicin in patients with vasomotor rhinitis' EUROPEAN ARCHIVES OF OTO-RHINO-LARYNGOLOGY vol. 248, no. 4, 1991, pages 191 - 194, XP000603518
- YAGI, Y. ET AL.: 'The difference in citric acid-induced cough in congenitally bronchial-hypersensitive (BHS) and bronchial hyposensitive (BHR) guinea pigs' EXPERIMENTAL ANIMALS vol. 50, no. 5, 2001, pages 371 - 378, XP055140586
- Jan M. Lundberg ET AL: "Capsaicin-induced desensitization of airway mucosa to cigarette smoke, mechanical and chemical irritants", Nature, vol. 302, no. 5905, 1 March 1983 (1983-03-01), pages 251-253, XP055670403, London ISSN: 0028-0836, DOI: 10.1038/302251a0
- T Kaneko ET AL: "Capsaicin reduces ozone-induced airway inflammation in guinea pigs.", AMERICAN JOURNAL OF RESPIRATORY AND CRITICAL CARE MEDICINE., vol. 150, no. 3, 1 September 1994 (1994-09-01), pages 724-728, XP055669404, US ISSN: 1073-449X, DOI: 10.1164/ajrccm.150.3.8087343
- B. F. Bessac ET AL: "Breathtaking TRP Channels: TRPA1 and TRPV1 in Airway Chemosensation and Reflex Control", PHYSIOLOGY, vol. 23, no. 6, 1 December 2008 (2008-12-01), pages 360-370, XP055322923, US ISSN: 1548-9213, DOI: 10.1152/physiol.00026.2008
- CHUNG K F ET AL: "Prevalence, pathogenesis, and causes of chronic cough", LANCET, ELSEVIER, AMSTERDAM, NL, vol. 371, no. 9621, 19 April 2008 (2008-04-19), pages 1364-1374, XP022615777, ISSN: 0140-6736, DOI: 10.1016/S0140-6736(08)60595-4 [retrieved on 2008-04-17]
- Yukihiro Yagi ET AL: "The Difference in Citric Acid-Induced Cough in Congenitally Bronchial-Hypersensitive (BHS) and Bronchial-Hyposensitive (BHR) Guinea Pigs.", Experimental Animals, vol. 50, no. 5, 1 January 2001 (2001-01-01), pages 371-378, XP055140586, ISSN: 1341-1357, DOI: 10.1538/expanim.50.371

## Description

### Field of the Invention

The present invention relates generally to a product for use in the treatment of cough.

### Description of the related art

Cough is an essential protective physiological mechanism to prevent food and liquid, dust, and chemicals to reach the lower airways, but it is also a symptom of many inflammatory diseases of the lungs. Coughing is one of the most common symptoms for which patients consult a doctor in the western world and current therapies are often unsatisfactory^{1,2}. Chronic cough is also clearly associated with significant social and psychological impacts^{3,4}. Patients with cough without any obvious explanation to the problem are often seen in clinical care and cough is arbitrarily defined as being chronic when it has lasted for more than 8 weeks⁵, even if the definition of chronic cough varies in the literature. Epidemiologic studies indicate that chronic cough is very prevalent in the community (up to 20% of the population) and could be increasing in relation to rising environmental pollution². In the present application such patients will be referred to as having chronic persistent unexplained cough. How common this condition is has also been debated^{5,6}. However, since there still are patients with cough for which no cure has been found, there is a need for a product (medicament) which may be used for treatment of cough and reduce or relieve coughing, e.g. to be used in treatment of patients having chronic persistent unexplained cough or increased cough reflex sensitivity.

The use of capsaicin for the treatment or prevention of bronchial diseases (especially asthma, (pseudo)croup and nose or throat inflammation) is described in DE 10 2004 063 363. In WO 2011 / 061 330 is described how capsaicin may be used in cough drops or throat lozenges and in DE 103 17 843 is disclosed that the addition of black pepper, comprising capsaicin, to animal food proved to reduce symptoms of colds such as coughing and runny nose.

Millqvist, The airway sensory hyperreactivity syndrome, Pulmonary Pharmacology & Therapeutics 24 (2011) 263-266 discloses that a suggested explanation for a group of patients suffering from unexplained chronic cough is a hyperreactivity of the sensory nerves of the entire airways, denoted sensory hyperreactivity (SHR).

Yagi et al., The difference in citric acid-induced cough in congenitally bronchial-hypersensitive (BHS) and bronchial hyposensitive (BHR) guinea pigs, Experimental Animals 50(5) (2001) 371-378 investigated cough elicitation and major physiological factors influencing cough occurrence in congenitally BHS and BHR guinea pigs exposed to citric acid aerosol. Salbutamol, atropine and capsaicin pre-treatments prolonged the cough latency in BHS, but only salbutamol prolonged the latency in BHR. After salbutamol pre-treatment all BHR guinea pigs exhibited cough, while 66.7% of BHS guinea pigs exhibited it. Vagal blocking by atropine suppressed coughing in both BHS and BHR. Only a small number of BHR guinea pigs and no BHR guinea pigs exhibited a cough response after capsaicin and lidocaine pre-treatment whereas many BHS guinea pigs still produced cough after such pre-treatment.

Lundberg et al., Capsaicin-induced desensitization of airway mucosa to cigarette smoke, mechanical and chemical irritants, Nature 302(5905) (1983) 251-253 reported that cigarette smoke as well as light mechanical or local irritation and vagal nerve stimulation induced a subepithelial oedema in the rat trachea and bronchial tree. Irritation of the respiratory tract mucosa seemed to activate capsaicin-sensitive neurones, which via local axon reflexes induced an interstitial oedema. Capsaicin pre-treatment induced a long-lasting desensitization of the airways to various exogenous and anaphylactic irritants.

Kaneko et al., Capsaicin reduces ozone-induced airway inflammation in guinea pigs, American Journal of Respiratory and Critical Care Medicine 150(3) (1994) 724-728 disclosed that capsaicin reduced but did not abolish the increase in airway vascular permeability induced by 3 ppm ozone for 30 min. Propranolol and atropine had no effect on airway vascular permeability. These results indicated that neuropeptides released from sensory nerves partly mediate ozone-induced airway inflammation, and that adrenergic and cholinergic pathways were not involved.

Bessac et al., Breathtaking TRP Channels: TRPA and TRPV1 in Airway Chemosensation and Reflex Control, Physiology 23(6) (2008) 360-370 revealed an essential role for TRPA1 in airway chemosensation and inflammation. TRPA1 is activated by chlorine, reactive oxygen species, and noxious constituents of smoke and smog, initiating irritation and airway reflex responses. Together with TRPV1, the capsaicin receptor, TRPA1 may contribute to chemical hypersensitivity, chronic cough, and airway inflammation in asthma, COPD, and reactive airway dysfunction syndrome.

### Summary of the invention

The present invention is defined by the independent claim. Further embodiments are defined by the dependent claims.

The invention relates to a food additive, pharmaceutical preparation for oral intake, for example formulated as a tablet, capsule, powder, syrup, drink or other suitable formulations that comprises capsaicin, the "hot" ingredient in chili pepper. More precisely the invention concerns a new application of orally taken capsaicin to relive and reduce coughing caused by other irritants then the capsaicin itself. We believe that we for the first time disclose the idea of using oral intake of low doses of capsaicin to ameliorate irritation based cough from other irritants then capsaicin.

The idea to use capsaicin as an active substance for treatment of cough is based on an extensive work in finding a treatment for cough. Since cough is a commonly occurring symptom around the world, a lot of efforts have been done in identifying causes and possible cures. When known causes for cough such as various infections, cancer, foreign body aspiration, cystic fibrosis, alveolitis, asthma, chronic obstructive pulmonary disease (COPD), medication with angiotensin converting enzyme (ACE) inhibitor, gastroesophageal reflux disease (GERD) or post-nasal drip syndrome have been excluded, still a group of patients with unexplained cough remains. A discrete clinical entity has been suggested for patients with such chronic cough in whom the cough is often triggered by environmental stimuli and, furthermore, in whom it initially may have developed after an upper respiratory tract infection ⁷. A similar group of patients with airway symptoms induced by environmental irritants report problems with coughing, chest discomfort, dyspnoea, rhinitis and eye irritation has been identified^{8,9}. The symptoms mimic asthma, but asthma-specific tests are negative. These patients have an increased cough reaction to inhaled capsaicin (the active compound of chili peppers), CAS nr 404-86-4, a tasteless and odorless substance that stimulates sensory nerves and reflects sensory nerve reactivity¹⁰. Such airway symptoms are interpreted as airway sensory hyperreactivity (SHR). Cigarette smoke, car exhaust and perfumed products are some of the triggers for SHR symptoms⁸. In most cases, these patients could also be diagnosed with chronic persistent unexplained cough⁶ or cough hypersensitivity syndrome¹¹. SHR affects more than 6% of the adult population in Sweden, mainly women, according to a population-based epidemiologic study¹² where SHR diagnosis was based on a questionnaire, the chemical sensitivity scale for sensory hyperreactivity (CSS-SHR), in combination with a standardized positive capsaicin inhalation provocation test^{13,14}.

Morice et al. have developed a questionnaire; the Hull Airway Reflux Questionnaire (HARQ) to identify coughers with a novel paradigm for understanding chronic cough¹⁵. This paradigm; the "Cough Hypersensitivity Syndrome" includes as well patients with symptoms that may indicate a reflux disease as patients with a general hypersensitivity towards e.g. environmental irritants. The patients are classified as having a cough hypersensitivity syndrome that also comprises both sensitivity to environmental irritants and augmented capsaicin cough answer^{11,16}.

In recent years there has been an emerging interest in the family of transient receptor potential (TRP) ion channels, which can be found in most of the human organ systems. They are able to sense temperature, noxious stimuli, pain, stretch, and osmolarity, among other factors. The main foci of such triggers in the airways are ion channels belonging to the transient receptor potential vanilloid (TRPV) and the transient receptor potential ankyrin (TRPA) families¹⁷⁻²⁰. The first genetically identified TRP receptor was the TRPV1, also called the capsaicin receptor, as capsaicin (8-Methyl-N-vanillyl-*trans*-6-nonenamide) is known to be an important stimulating factor²¹. Nociceptive sensory neurons also take part in protective reflexes, including the cough and sneeze reflexes, and release inflammatory neuropeptides in the periphery upon stimulation by different environmental stimuli. Patients with chronic cough had an increase of TRPV1-staining nerve profiles and, furthermore, a significant correlation between capsaicin tussive response and the number of TRPV1-positive nerves^{22,23}. Several studies have shown that patients with chronic cough have increased capsaicin cough sensitivity^{11,16}. The results from all these studies suggest that the pathophysiology of chronic persistent unexplained cough is related to airway mucosal TRP receptors on sensory nerves, as well reacting to noxious stimuli¹⁹.

Also in asthma, rhinitis and COPD, airway symptoms induced by environmental irritants are common. These patients often complain of symptoms induced by exposure to cold air, smoke, exhaust fumes, strong odorants, and exercise. Although the role of sensory nerves in airway inflammation and obstruction is controversial, there is a growing body of evidence that sensory nerves mediate many of the symptoms in these patients²⁴. In line with this, patients with asthma/COPD and allergic rhinitis exhibit, respectively, an exaggerated cough and secretory response to the sensory nerve stimulant capsaicin^{11,16,25}. The link between the inflammatory cascade of asthma, causing bronchial hyperreactivity, and the axon reflex of bronchoconstriction is although intensive research still not clarified²⁶. Increasing evidence points to a potential role for members of the TRP family of cation channels on airway sensory non-adrenergic non-cholinergic (e-NANC) nerves in the development of bronchial hyperreactivity and several features of asthmatic disease^{19,26,27}.

### Capsaicin in food

Capsaicin is found naturally in a great variety of food dishes comprising different kinds of chili products and gives a "hot" taste. The use of chili in food varies between different countries and cultures. Most western countries have no long tradition of the use of chili in cooking. A dish with a lot of hot chili can result in undesired symptoms like irritation in the mouth and throat, sneezing, eye irritation and sometimes coughing. It is "common knowledge" that it is possible to get used to spicy food by gradually increase the intake. The more you eat, the fewer symptoms you get. The scientific explanation to this phenomenon is on receptor level. The TRPV1 receptors, which are activated by capsaicin use neuropeptides to evoke brain signals and if you regularly stimulate these receptors with capsaicin rich food the neuropeptides are depleted and no or few symptoms are awaked by spicy food.

### Pain and TRPV1

The TRPV1 receptor is also known to react to pain stimuli and mirror pain sensitivity ²⁸. For a long time, different pain conditions (arthritis, cystitis, human immunodeficiency virus, and diabetic neuropathy) have been treated with topically applied capsaicin (crèmes, gels and patches)^{28,29}. A recently developed site-specific capsaicin therapy with high-dose patches and injectable preparations seem to be safe and reportedly provide long-lasting analgesia with rapid onset³⁰.

### Gastrointestinal symptoms and TRPV1

In the gastrointestinal system the TRPV1 receptors are widely represented and some researchers make a connection between these receptors and chronic symptoms like irritable bowl syndrome (IBS) and have shown increased expression of TRPV1 receptors in this condition³¹. Recent studies show improvement of IBS and gut pain sensitivity after regular intake of capsaicin powder³²⁻³⁷.

### Cough and TRPV1

Capsaicin provokes cough when inhaled via the TRPV1 receptors and has for several years been used in the study of cough and is regarded to be to be an extremely safe research tool with no serious adverse reactions reported over the past 2 decades³⁸. The capsaicin cough sensitivity increases during airway infections and is also heightened in different well-defined pulmonary conditions like asthma, COPD and pulmonary fibrosis^{8,39-42}. In chronic cough without evident medical explanation and in SHR there is remarkably increased cough sensitivity to inhaled capsaicin^{8,11,43}.

In summary, testing with inhaled capsaicin is a well-documented tool in the diagnostics of chronic cough induced by environmental irritants where no evident other explanation to the cough, like asthma or allergy, has been found. The present invention is based on the idea that capsaicin could be used not only as an indicator but as a cure for cough which could be used for a down-regulation of coughing following a regular consumption of a capsaicin product. Patients with chronic persistent unexplained cough will probably benefit most from the invention but also in rhinitis and other conditions known to have cough symptoms and increased cough reflex sensitivity the invention can improve symptoms.

A first object of the invention is to treat patients with chronic persistent unexplained cough, using a regular and oral administration of a product containing capsaicin.

Another object of the invention is to provide a food additive, pharmaceutical preparation oral intake, for example formulated as a tablet, capsule, powder, syrup, drink or other suitable formulations that comprises capsaicin .

### Detailed description of the invention and preferred embodiments thereof

Capsaicin is found naturally in a great variety of food dishes comprising different kinds of chili products and gives a "hot". The invention herein concerns a new oral application for a capsaicin product to relive and reduce coughing related to other irritants then the capsaicin itself. Cough is an essential protective physiological mechanism to prevent food and liquid, dust, and chemicals to reach the lower airways, but it is also a symptom of many inflammatory diseases of the lungs. Patients with cough without any obvious explanation to the problem are often seen in clinical care and cough is arbitrarily defined as being chronic when it has lasted for more than 8 weeks. In the present application such patients will be referred to as having chronic persistent unexplained cough.

Capsaicin is known to induce cough. To the contrary of present knowledge we have found and made the invention herein that relates to a down-regulation of coughing following a regular consumption of a food additive, pharmaceutical preparation for oral intake, for example formulated as a tablet, capsule, powder, syrup, drink or other suitable formulations that comprises capsaicin. Patients with chronic persistent unexplained cough will probably benefit most from the invention but also in rhinitis and other conditions known to have cough symptoms and increased cough reflex sensitivity the invention has a potential to improve symptoms.

The capsaicin should be administered orally in low doses to induce desensitisation also against other irritants than capsaicin itself.

Chronic cough without evident medical explanations and also the cough component in sensory hyperreactivity (SHR) depend on an up regulation of the TRPV1 receptor system on airway sensory nerves. In parallel with the use of capsaicin in other conditions to improve for example pain and gastrointestinal symptoms, regular intake of a food additive comprising capsaicin (chili powder) will decrease the cough reflex sensitivity and improve cough symptoms by a continuous depletion of neuropeptides. Also other non-allergic airway and mucosal symptoms without evident cause may benefit from capsaicin, e.g. rhinitis, asthma-like symptoms, non-allergic eye irritation and burning moth syndrome. This may be the cause also in more general symptoms, from chemical and environmental sensitivity.

The administration of the product could be oral but also local by gargling, chewing of tablets or gums, or spraying, nasal or bronchial inhaling of aerosols or powder.

Capsaicin (8-methyl-n-vanillyl- 6-nonenamide, Chemical Abstracts Service (CAS) registry number is 404-86-4) as used herein may comprise pure capsaicin, and may also be in diluted form, calculated to be used in equivalent strength, such as chili pepper powder or other powders. By equivalent to capsaicin is thus meant a calculated strength firstly based on the form of capsaicinoid, such as dihydrocapsaicin, nordihydrocapsaicin, homodihydrocapsaicin, homocapsaicin, according to Table 1 and secondly if the active material is from a source which is not pure capsaicinoid such as chili-powder (which typically contains 0,4% pure capsaicin) and thirdly if also such material as chili-powder is diluted with a filler. For example if undiluted chili-powder is used in a product of the invention which is targeted at a 0,4 mg capsaicin level, the amount of chili-powder to get to the equivalent strength is 100 mg.

**Table 1: Examples of capsaicinoids**

| **Capsaicinoid name** | **Abbrev.** | **Typical equivalent strengh** | **Scoville heat units** | **Chemical structure** |
|---|---|---|---|---|
| Capsaicin | C | 100% | 16,000,000 | 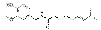 |
| Dihydrocapsaicin | DHC | 32% | 16,000,000 | 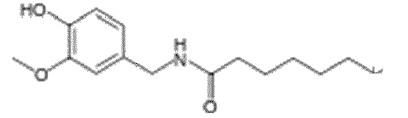 |
| Nordihydrocapsaicin | NDHC | 10% | 9,100,000 | 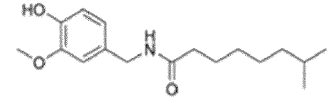 |
| Homodihydrocapsaicin | HDHC | 1,5% | 8,600,000 | 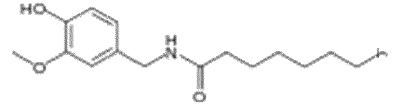 |
| Homocapsaicin | HC | 1,5% | 8,600,000 | 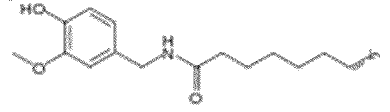 |

Preferably the daily doses of pure capsaicin, or equivalent, may vary from 0.01 mg to 100 mg, more preferably from 0.05 mg to 10 mg and most preferably from 0.2 mg 4 mg. The daily doses may be distributed one or several times a day, preferably between 1 to 5 times a day, commonly between 2 - 3 times a day. The daily doses is preferably distributed as unit dosages wherein each unit dosage may have a content of pure capsaicin, or equivalent, from 0.01 mg to 20 mg, more preferably from 0.05 to 5 mg and most preferably from 0.1 to 2 mg.

### EXAMPLE 1

Natural chili pepper powder in capsules. The capsules are made from pectin that easily is digested in the stomach. Each active capsule comprises 100 mg chili powder of which 0.4% (0.4 mg) is a capsaicin compound. (Heinrich Klenk GmbH & CO KG, Germany) from chili powder, which is certified by European standard; European Pharmacopeia V; 2005 Cayennepepper, Capsici fructus, p.1662-1663. The powder is made from natural milled chili pepper and comprises 0.4% chili as capsaicin, dihydrocapsaicin and nordihydrocapsaicin.

The capsaicin in the capsules may also be pure capsaicin extracted from natural chilli fruits (Sigma-Aldrich, Sweden AB, Stockholm, M2028). Each active capsule will then comprise 0,4 mg pure capsaicin together with a filler material (cellulosa or sugar). Each capsule will then contain 100 mg (0,04 mg pure capsaicin together with 99,96 mg filling material.

### EXAMPLE 2

The aim was to study whether a regular, standardized oral intake of natural capsaicin (chili powder) equal to a diet with very spicy food can improve cough symptoms and decrease cough reflex sensitivity measured by a standardized capsaicin inhalation cough test.

### Study group

25 consecutively selected patients, having chronic refractory unexplained cough and claiming sensitivity to environmental irritants like perfumed products and chemicals participate together with 25 healthy control subjects. None of the participants have any other pulmonary or respiratory illness like allergy, asthma or chronic obstructive pulmonary disease (COPD).

### Study design

Each participant visits the asthma and allergy clinic at Sahlgrenska University Hospital in Gothenburg, Sweden 3 times during a 8 weeks study period before the start of the study and after 4 and 8 weeks. At each visit cough sensitivity is assessed by a standardized capsaicin inhalation cough test¹⁴. During the 8 weeks the participants daily fill in a "patient symptom diary". During 4 weeks the participants take active capsaicin food additive capsules (chili powder) and during one month placebo capsules. The order of this is cross over, randomized and double blind.

**Table 2: Flow scheme**

| Baseline visit all participants | 2 weeks | 2weeks | Half time visit all participants Cross-over | 2 weeks | 2 weeks | End visit all participants |
|---|---|---|---|---|---|---|
| **Group A** 12 pat + 13 cont | Capsaicin 0.4 mg, 1 x 2 | Capsaicin 0.4 mg 2x2 | Group A | Placebo 1 x 2 | Placebo 2x2 | Group A |
| Capsaicin test Questionnaires all participants | Symptomdiary | Symptomdiary | Capsaicin test Questionnaires all participants | Symptomdiary | Symptomdiary | Capsaicin test Questionnaires all participants |
| **Group B** 13 pat + 12 cont | Placebo | Placebo | Group B | Capsaicin 0.4 mg 1 x 2 | Capsaicin 0.4 mg | Group B |

### The food additive

As per Example 1. During the active treatment period, the first two weeks, the participants take 1 capsule morning and evening and during the next two weeks 2 capsules morning and evening. All capsules are taken in connection to a meal.

### Questionnaires

- Local questionnaire regarding general health and airway sensitivity to chemicals and scents
- The CSS-SHR (chemical sensitivity scale sensory hyperreactivity): estimating social and emotional influence from chemicals and scents
- The Hull cough questionnaire
- Symptom diaries for 8 weeks

### Patients - inclusion criteria

- Age 18-70 years
- Airway sensitivity from chemicals and scents
- Daily cough symptoms
- Positive capsaicin inhalation cough test
- No gastrointestinal symptoms or problems
- Otherwise healthy
- Not pregnant or breast feeding
- No airway infection the last 4 weeks

### Results

The capsaicin cough thresholds are significantly heightened. The daily symptom diaries show gradually significant improvement in all patients with 16 of them having no cough symptoms at all during the last week of active treatment. In patients starting with active treatment during 4 weeks the cough symptoms return after 2 placebo weeks and also the capsaicin cough thresholds are significantly lowered (impaired) after 4 placebo weeks compared to 4 weeks with active treatment.

### REFERENCES

1. Irwin RS, Curley FJ, French CL. Chronic cough. The spectrum and frequency of causes, key components of the diagnostic evaluation, and outcome of specific therapy. Am Rev Respir Dis 1990;141:640-7.
2. Chung KF, Pavord ID. Prevalence, pathogenesis, and causes of chronic cough. Lancet 2008;371:1364-74.
3. French CL, Irwin RS, Curley FJ, Krikorian CJ. Impact of chronic cough on quality of life. Arch Intern Med 1998;158:1657-61.
4. Young EC, Smith JA. Quality of life in patients with chronic cough. Ther Adv Respir Dis 2010;4:49-55.
5. Morice AH, Fontana GA, Sovijarvi AR, Pistolesi M, Chung KF, Widdicombe J, O'Connell F, Geppetti P, Gronke L, De Jongste J, Belvisi M, Dicpinigaitis P, Fischer A, McGarvey L, Fokkens WJ, Kastelik J. The diagnosis and management of chronic cough. Eur Respir J 2004;24:481-92.
6. Dicpinigaitis PV. Cough: an unmet clinical need. Br J Pharmacol 2011;163:116-24.
7. Haque RA, Usmani OS, Barnes PJ. Chronic idiopathic cough: a discrete clinical entity? Chest 2005;127:1710-3.
8. Millqvist E, Bende M, Lowhagen O. Sensory hyperreactivity - a possible mechanism underlying cough and asthma-like symptoms. Allergy 1998;53:1208-12.
9. Ternesten-Hasseus E, Lowhagen O, Millqvist E. Quality of life and capsaicin sensitivity in patients with airway symptoms induced by chemicals and scents: a longitudinal study. Environ Health Perspect 2007;115:425-9.
10. Clapham DE. TRP channels as cellular sensors. Nature 2003;426:517-24.
11. Morice AH, Faruqi S, Wright CE, Thompson R, Bland JM. Cough hypersensitivity syndrome: a distinct clinical entity. Lung 2011;189:73-9.
12. Johansson A, Millqvist E, Nordin S, Bende M. Relationship between self-reported odor intolerance and sensitivity to inhaled capsaicin: proposed definition of airway sensory hyperreactivity and estimation of its prevalence. Chest 2006;129:1623-8.
13. Nordin S, Millqvist E, Lowhagen O, Bende M. A short chemical sensitivity scale for assessment of airway sensory hyperreactivity. Int Arch Occup Environ Health 2004;77:249-54.
14. Johansson A, Lowhagen O, Millqvist E, Bende M. Capsaicin inhalation test for identification of sensory hyperreactivity. Respir Med 2002;96:731-5.
15. Morice AH. The Cough Hypersensitivity Syndrome: A Novel Paradigm for Understanding Cough. Lung 2009;188:87-90.
16. Millqvist E. The airway sensory hyperreactivity syndrome. Pulm Pharmacol Ther 2011;24:263-6.
17. Nilius B. TRP channels in disease. Biochim Biophys Acta 2007;1772:805-12.
18. Venkatachalam K, Montell C. TRP channels. Annual review of biochemistry 2007;76:387-417.
19. Bessac BF, Jordt SE. Breathtaking TRP Channels: TRPA1 and TRPV1 in Airway Chemosensation and Reflex Control. Physiology (Bethesda) 2008;23:360-70.
20. Birrell MA, Belvisi MG, Grace M, Sadofsky L, Faruqi S, Hele DJ, Maher SA, Freund-Michel V, Morice AH. TRPA1 agonists evoke coughing in guinea pig and human volunteers. Am J Respir Crit Care Med 2009;180:1042-7.
21. Tominaga M, Caterina MJ, Malmberg AB, Rosen TA, Gilbert H, Skinner K, Raumann BE, Basbaum AI, Julius D. The cloned capsaicin receptor integrates multiple pain-producing stimuli. Neuron 1998;21:531-43.
22. Groneberg DA, Niimi A, Dinh QT, Cosio B, Hew M, Fischer A, Chung KF. Increased expression of transient receptor potential vanilloid-1 in airway nerves of chronic cough. Am J Respir Crit Care Med 2004;170:1276-80.
23. Mitchell JE, Campbell AP, New NE, Sadofsky LR, Kastelik JA, Mulrennan SA, Compton SJ, Morice AH. Expression and characterization of the intracellular vanilloid receptor (TRPV1) in bronchi from patients with chronic cough. Experimental lung research 2005;31:295-306.
24. Sarin S, Undem B, Sanico A, Togias A. The role of the nervous system in rhinitis. J Allergy Clin Immunol 2006;118:999-1016.
25. Sanico AM, Koliatsos VE, Stanisz AM, Bienenstock J, Togias A. Neural hyperresponsiveness and nerve growth factor in allergic rhinitis. Int Arch Allergy Immunol 1999;118:154-8.
26. Veres TZ, Rochlitzer S, Braun A. The role of neuro-immune cross-talk in the regulation of inflammation and remodelling in asthma. Pharmacol Ther 2009;122:203-14.
27. Colsoul B, Nilius B, Vennekens R. On the putative role of transient receptor potential cation channels in asthma. Clin Exp Allergy 2009;39:1456-66.
28. Caterina MJ, Julius D. The vanilloid receptor: a molecular gateway to the pain pathway. Annu Rev Neurosci 2001;24:487-517.
29. Robbins W. Clinical applications of capsaicinoids. Clin J Pain 2000;16:S86-9.
30. Knotkova H, Pappagallo M, Szallasi A. Capsaicin (TRPV1 Agonist) therapy for pain relief: farewell or revival? Clin J Pain 2008;24:142-54.
31. Geppetti P, Trevisani M. Activation and sensitisation of the vanilloid receptor: role in gastrointestinal inflammation and function. Br J Pharmacol 2004;141:1313-20.
32. Bortolotti M, Coccia G, Grossi G. Red pepper and functional dyspepsia. N Engl J Med 2002;346:947-8.
33. Fuhrer M, Hammer J. Effect of repeated, long term capsaicin ingestion on intestinal chemo- and mechanosensation in healthy volunteers. Neurogastroenterol Motil 2009;21:521-7, e7.
34. Gonlachanvit S. Are rice and spicy diet good for functional gastrointestinal disorders? JNeurogastroenterol Motil 2010;16:131-8.
35. Gonlachanvit S, Fongkam P, Wittayalertpanya S, Kullavanijaya P. Red chili induces rectal hypersensitivity in healthy humans: possible role of 5HT-3 receptors on capsaicin-sensitive visceral nociceptive pathways. Aliment Pharmacol Ther 2007;26:617-25.
36. Gonlachanvit S, Mahayosnond A, Kullavanijaya P. Effects of chili on postprandial gastrointestinal symptoms in diarrhoea predominant irritable bowel syndrome: evidence for capsaicin-sensitive visceral nociception hypersensitivity. Neurogastroenterol Motil 2009;21:23-32.
37. Hammer J. Effect of repeated capsaicin ingestion on intestinal chemosensation and mechanosensation. Aliment Pharmacol Ther 2006;24:679-86.
38. Dicpinigaitis PV, Alva RV. Safety of capsaicin cough challenge testing. Chest 2005;128:196-202.
39. Dicpinigaitis PV. Capsaicin responsiveness in asthma and COPD. Thorax 2001;56:162.
40. Doherty MJ, Mister R, Pearson MG, Calverley PM. Capsaicin responsiveness and cough in asthma and chronic obstructive pulmonary disease. Thorax 2000;55:643-9.
41. Weinfeld D, Ternesten-Hasseus E, Lowhagen O, Millqvist E. Capsaicin cough sensitivity in allergic asthmatic patients increases during the birch pollen season. Ann Allergy Asthma Immunol 2002;89:419-24.
42. Ekstrand Y, Ternesten-Hasseus E, Arvidsson M, Lofdahl K, Palmqvist M, Millqvist E. Sensitivity to Environmental Irritants and Capsaicin Cough Reaction in Patients with a Positive Methacholine Provocation Test before and after Treatment with Inhaled Corticosteroids. J Asthma 2011;48:482-9.
43. Gibson PG, Ryan NM. Cough pharmacotherapy: current and future status. Expert Opin Pharmacother 2011;12:1745-55.

## Claims

1. A product containing capsaicin for use in the treatment of chronic persistent unexplained cough or increased cough reflex sensitivity, wherein said product is in a unit dosage form which comprises a content of pure capsaicin from 0.05 to 5 mg, and wherein the product is administered orally.

2. A product for use according to claim 1, wherein said unit dosage form comprises a content of pure capsaicin from 0.1 to 2 mg.

3. A product for use according to claims 1 or 2, wherein said product is in the form of a tablet, capsule, powder, syrup or drink.

## Patentansprüche

1. Produkt, das Capsaicin enthält, zur Verwendung bei der Behandlung von chronisch anhaltendem, ungeklärtem Husten oder erhöhter Hustenreflexsensibilität, wobei das Produkt in einer Einheitsdosierungsform vorliegt, die einen Gehalt an reinem Capsaicin von 0,05 bis 5 mg umfasst, und wobei das Produkt oral verabreicht wird.

2. Produkt zur Verwendung nach Anspruch 1, wobei die Einheitsdosierungsform einen Gehalt an reinem Capsaicin von 0,1 bis 2 mg umfasst.

3. Produkt zur Verwendung nach Anspruch 1 oder 2, wobei das Produkt in Form einer Tablette, einer Kapsel, eines Pulvers, eines Sirups oder eines Getränks vorliegt.

## Revendications

1. Produit contenant de la capsaïcine à utiliser dans le traitement de la toux chronique persistante inexpliquée ou de la sensibilité accrue des réflexes de toux, dans lequel ledit produit est sous une forme posologique unitaire qui comprend une teneur en capsaïcine pure de 0,05 à 5 mg, et dans lequel le produit est administré par voie orale.

2. Produit à utiliser selon la revendication 1, dans lequel ladite forme posologique unitaire comprend une teneur en capsaïcine pure de 0,1 à 2 mg.

3. Produit à utiliser selon les revendications 1 ou 2, dans lequel ledit produit se présente sous la forme d'un comprimé, d'une capsule, d'une poudre, d'un sirop ou d'une boisson.
